(19) 

Europäisches Patentamt

European Patent Office

Office européen des brevets

(11) **EP 2 705 791 B1**

(12) **EUROPEAN PATENT SPECIFICATION**

(45) Date of publication and mention of the grant of the patent:
**28.02.2018 Bulletin 2018/09**

(51) Int Cl.:
*A61B 5/11* (2006.01)      *A61B 5/00* (2006.01)
*A61B 5/0402* (2006.01)

(21) Application number: **13183562.1**

(22) Date of filing: **09.09.2013**

(54) **A device and method for estimating energy expenditure during exercise**

Vorrichtung und Verfahren zur Schätzung des Energieverbrauchs während einer Übung

Dispositif et procédé d'estimation de dépenses en énergie durant un exercice

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **07.09.2012 GB 201216014**

(43) Date of publication of application:
**12.03.2014 Bulletin 2014/11**

(73) Proprietor: **Toumaz Healthcare Limited Abingdon Oxfordshire OX14 4RZ (GB)**

(72) Inventors:
• **Hernandez-Silveira, Miguel Abingdon, Oxfordshire OX14 4RZ (GB)**
• **Ang, Su-Shin Abingdon, Oxfordshire OX14 4RZ (GB)**

(74) Representative: **Branderhorst, Matthijs Pieter Arie et al Marks & Clerk LLP Fletcher House Heatley Road The Oxford Science Park Oxford OX4 4GE (GB)**

(56) References cited:
EP-A1- 1 618 844      WO-A2-2007/048431
DE-A1-102009 044 612      US-A1- 2001 020 135

US-A1- 2004 186 390

• A Ardévol ET AL: "During intense exercise, obese women rely more than lean women on aerobic energy", Pflügers Archiv - European Journal of Physiology, 1 February 1998 (1998-02-01), pages 495-502, XP055092100, DOI: 10.1007/s004240050544 Retrieved from the Internet: URL:http://rd.springer.com/content/pdf/10.1007/s004240050544.pdf [retrieved on 2013-12-06]

• SCOTT CHRISTOPHER B ET AL: "Energy expenditure before, during, and after the bench press.", JOURNAL OF STRENGTH AND CONDITIONING RESEARCH / NATIONAL STRENGTH & CONDITIONING ASSOCIATION MAR 2009, vol. 23, no. 2, March 2009 (2009-03), pages 611-618, XP008166204, ISSN: 1533-4287

• JUMAH ET AL: "Analyse und Vergleich von Methoden zur Bestimmung der anaeroben Kapazität und Leistungsfähigkeit bei Freizeitsportlern auf dem Fahrradergometer", 1 October 2009 (2009-10-01), DISSERTATION FAKULTAET FUER MEDIZIN DER TECHNISCHEN UNIVERSITAETMUENCHEN ZUR ERLANGUNG DES AKADEMISCHEN GRADES EINES DOKTORS DERMEDIZIN, XX, XX, PAGE(S) 1 - 90, XP002625372, * page 8 - page 26 *

• BEAVER W L ET AL: "Improved detection of lactate threshold during exercise using a log-log transformation", JOURNAL OF APPLIED PHYSIOLOGY,, vol. 59, no. 6, 1 December 1985 (1985-12-01), pages 1936-1940, XP008185071,

EP 2 705 791 B1

## Description

Field of the Invention

The present invention relates to a device and method for estimating energy expenditure during exercise. It is of particular use in providing a more accurate estimate of energy expenditure when exercising above the anaerobic threshold.

Background of the Invention

There are three sub-systems in the human body that are responsible for the release of energy: aerobic metabolism, alactic anaerobic metabolism (phosphagen system), and anaerobic lactic metabolism. In all of these systems, energy release is achieved through the breakdown of Adenosine Triphosphate (ATP) into Adenosine Diphosphate (ADP). These systems differ in the type of biochemical reactions that are used in the production of ATP, and the efficiency of ATP production.

When the physical workload placed on a subject is gradually increased from rest to strenuous activities, ATP stored within the muscles is utilized for the initial release of energy by means of alactic anaerobic metabolism. Small amounts of energy are produced by anaerobic lactic metabolism, with lactic acid (lactate) as the by-product. However, the lactate produced by alactic metabolism is buffered by bicarbonate in the bloodstream, leading to an increased rate of carbon dioxide production, without a reduction in the blood pH. The rate of oxygen uptake increases proportionally with the rate of carbon dioxide production.

Upon depletion of the ATP stored within the muscles, which occurs after a very short period of time, aerobic metabolism becomes the dominant source of energy release. In aerobic metabolism glucose is efficiently converted into ATP for further energy release.

When the workload is increased further there is insufficient oxygen in the blood to support aerobic metabolism and, therefore, anaerobic lactic metabolism overtakes aerobic metabolism as the dominant system for producing ATP. Anaerobic lactic metabolism is relatively inefficient at producing ATP molecules from glucose, producing 2 molecules of ATP for every molecule of glucose compared to aerobic metabolism producing 38 molecules of ATP for every molecule of glucose. Additionally, the lactic acid produced by the anaerobic respiration cannot be completely buffered within the blood leading to an increased rate of carbon dioxide release relative to oxygen uptake by the body. The build-up of lactate in the blood eventually leads to fatigue and breathlessness. The point at which lactate begins to build in a person's blood is known as the anaerobic threshold.

The efficiency of the energy release in the body can therefore be seen to differ depending on whether a subject is carrying out predominantly aerobic metabolism (below the anaerobic threshold) or predominantly anaerobic metabolism (above the anaerobic threshold) due to differences in efficiencies between the different energy sub-systems.

For example, compared to a highly trained athlete, a typically sedentary individual is likely to exceed their anaerobic threshold earlier and at lighter workloads. Consequently, even for the same workload, the energy expenditure by a sedentary individual will be greater than that of a highly trained athlete. This is because energy expenditure beyond the anaerobic threshold is greater than the energy expenditure below the anaerobic threshold due to the additional stress placed on the cardiovascular and respiratory systems for carbon dioxide removal.

United States Patent 6554776 describes a cardiopulmonary weight-loss system that computes the energy expenditure as a linear function of oxygen uptake and carbon dioxide production, as well as the point at which the anaerobic threshold has been exceeded. This aim of the system is to enhance weight-loss by maximizing fat burning. Fat burning is maximal during aerobic respiration and, thus, the system encourages users to stay beneath the anaerobic threshold and only calculates energy expenditure using an aerobic respiration model.

United States Patent 7470234 describes a portable device that monitors the vital signs of the subject, including the calorie expenditure, the real-time heart rate, and the anaerobic threshold to allow an individual to track their exercise. Energy expenditure is estimated by means of the intensity of the exercise and is calculated using an aerobic metabolism model. The anaerobic threshold is provided to a user to enable them to train within their anaerobic threshold zone.

United States Patent 7648463 describes eyewear that makes use of an optical sensor to detect the flow of blood, and correspondingly calculate the heart rate of the subject. The energy expenditure is then determined as a function of the heart rate assuming that the user is using aerobic metabolism.

A. Ardevol et al. European Journal of Physiology, 1 Feb. 1998, pages 495-502, which is regarded as the closest prior art to the present invention, describes how during intense exercise, obese women rely more than lean women on aerobic energy.

US 2004/186390 A1 describes a respiratory analyzer having a flow module which comprises a flow tube through which the flow rate of gases is determined using a flow rate meter and a computation module.

EP 1618844 A1 describes the determination of exercise intensity, which also involves classifying exercises according to activity level.

Summary of the Invention

The invention provides a device and a method as set out in the accompanying claims. Further herein disclosed is a device comprising an input configured to receive data from a sensor, an aerobic processor configured to estimate energy expenditure from the data using an aerobic metabolism model, an anaerobic threshold module configured to determine from the data whether the anaerobic threshold for the user has been exceeded, an output to output the estimated physical activity intensity if the anaerobic threshold for the user has not been exceeded, an anaerobic energy expenditure estimator configured to estimate energy expenditure due to anaerobic respiration from the data, a processor configured to combine the estimated anaerobic energy expenditure and the estimated aerobic energy expenditure to produce a total energy expenditure when the anaerobic threshold for the user has been exceeded, and an output to output the total energy expenditure if the anaerobic threshold for the user has been exceeded. By applying different models to calculate energy expenditure depending on whether the user is operating above or below their anaerobic threshold the user can be provided with a more accurate estimate of their energy expenditure.

The aerobic processor includes an exercise intensity classifier configured to classify the exercise of the user using data received by the input; and an estimator to estimate physical activity intensity and the estimated oxygen level in the respiratory gases according to the classification of the exercise wherein the output outputs the estimated physical activity intensity if the anaerobic threshold for the user has not been exceeded. By classifying the intensity of the exercise prior to estimating the physical activity intensity the most appropriate model for calculating energy expenditure due to aerobic respiration may be selected.

The anaerobic energy expenditure estimator includes a lactate estimator to estimate plasma lactate concentration; and a lactate to calorie convertor to convert the estimated plasma lactate concentration to a calorie value representing the anaerobic energy. The device further comprises an aerobic energy expenditure estimator configured to calculate a value representing the aerobic energy expenditure using the estimated oxygen level in the respiratory gases and a processor configured to add the calorie value representing the anaerobic energy and the calorie value representing the aerobic energy expenditure to provide an estimate of the total energy expenditure when the anaerobic threshold has been passed.

The plasma lactate concentration is estimated using an existing method:

$$LA = 10^{(0.082 \lg VO_2 - 0.2)} \text{ where } VO_2 < 1.51$$

$$LA = 10^{(2.88 \lg VO_2 - 0.7)} \text{ where } VO_2 \geq 1.51$$

where LA is the plasma lactate concentration.

The estimated plasma lactate concentration may be converted to a calorie value representing the energy used during anaerobic respiration using the equation:

$$PAI_{anaerobic} = \frac{20 LA}{0.75}$$

The anaerobic energy expenditure estimator calculates the calories using the following equation:

$$PAI_{aerobic} = 5.01 * VO_2$$

The sensor may be either one or a combination of any two or more physiological and/or biomechanical sensors. The sensors may be, for example, an ECG sensor, accelerometer and gyroscope.

The data may be one or more of the following parameters: heart rate, respiration rate, heart rate variability such as fluctuations of ECG R-R intervals, accelerometer measurements such as accelerometer activity counts, step counts and linear and angular accelerations and gyroscope measurements such as angular speed and/or distance.

The device input may be arranged to receive the data over a wireless connection or over a physical connection. Further herein disclosed is a method comprising receiving data from a body sensor, estimating energy expenditure using an aerobic metabolism model, determining from the data whether the anaerobic threshold for the user has been exceeded, outputting the estimated energy expenditure if the anaerobic threshold for the user has not been exceeded, estimating energy expenditure due to anaerobic respiration, combining the estimated anaerobic energy expenditure and the estimate aerobic energy expenditure to produce a total energy expenditure when the anaerobic threshold for the user has been exceeded and outputting the total energy expenditure if the anaerobic threshold for the user has been exceeded.

Estimating energy expenditure using an aerobic metabolism model includes classifying the exercise of the user using data received by the input and estimating physical activity intensity and the estimated oxygen level in the respiratory gases according to the classification of the exercise wherein the output outputs the estimated physical activity intensity if the anaerobic threshold for the user has not been exceeded.

Estimating energy expenditure due to anaerobic respiration includes estimating plasma lactate concentration and converting the estimated plasma lactate concentration to a calorie value representing the anaerobic energy. The method also includes calculating a calorie value representing the aerobic energy expenditure using the estimated oxygen level in the respiratory gases and adding the calorie value representing the anaerobic energy and the calorie value representing the aerobic energy expenditure to provide an estimate of the total energy expenditure when the anaerobic threshold has been passed.

The plasma lactate concentration is estimated using for example an existing method

$$LA = 10^{(0.082\,\lg VO_2 - 0.2)} \text{ where } VO_2 < 1.51$$

$$LA = 10^{(2.88\,\lg VO_2 - 0.7)} \text{ where } VO_2 \geq 1.51$$

where LA is the plasma lactate concentration.

Converting the estimated plasma lactate concentration to a calorie value representing the anaerobic energy may use the equation:

$$PAI_{anaerobic} = \frac{20 LA}{0.75}$$

Calculating a calorie value representing the aerobic energy expenditure may use the following equation:

$$PAI_{aerobic} = 5.01 * VO_2$$

The data may be one or more of the following parameters: heart rate, respiration rate, heart rate variability such as fluctuations of ECG R-R intervals, accelerometer measurements such as accelerometer activity counts, step counts and linear and angular accelerations and gyroscope measurements such as angular speed and/or distance.

Brief Description of the Drawings

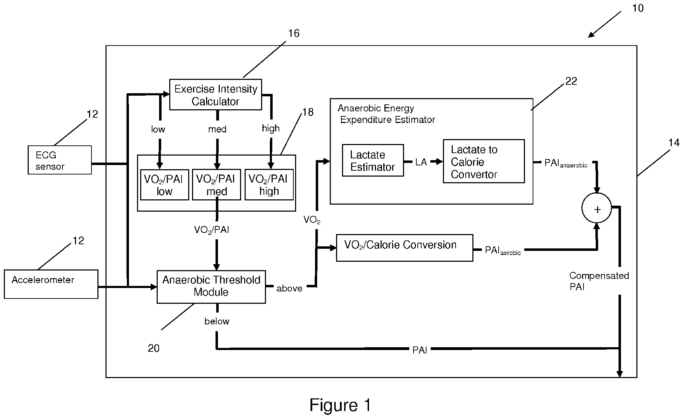

Figure 1 illustrates a device according to the present invention.

Detailed Description

A device 10 for calculating energy expenditure is illustrated in Figure 1. The device 10 includes sensors 12 such as an ECG sensor and an accelerometer, a processor 14 and a display (not shown).

The ECG sensor and accelerometer are attached to the user's body using any suitable means. The processor 14 receives an input from each of the sensors 12. The sensor input is passed to an exercise intensity classifier 16 which uses the input from one or more of the sensors 12 to classify the current activity intensity as "low", "moderate" or "high". For example, the exercise intensity classifier 16 may receive a heart rate and heart rate variability characteristics from an ECG sensor and use these values to calculate the exercise intensity in accordance with any suitable method.

The activity intensity classification is relayed to a Physical Activity Intensity and $VO_2$ estimator 18 where $VO_2$ is the estimated oxygen level in the respiratory gases. The Physical Activity Intensity and $VO_2$ estimator 18 estimates the Physical Activity Intensity and $VO_2$ of the user using the input from the one or more sensors 12 and relationships between the sensor inputs and the Physical Activity Intensity and $VO_2$. The relationships between the sensor inputs and the Physical Activity Intensity and $VO_2$ have been empirically derived using aerobic metabolism models for each of the different activity intensities. Thus, the Physical Activity Intensity value provided by the estimator is a value for the Physical Activity Intensity for aerobic metabolism at a given activity intensity.

Once the physical activity intensity and $VO_2$ values have been estimated they are passed to an anaerobic threshold module 20. The anaerobic threshold module 20 determines whether the anaerobic threshold for the user of the device has been exceeded or not.

For example, the anaerobic threshold module may determine if the anaerobic threshold has been exceeded based on the extent of variation in the heart rate variability (which may be received from the ECG sensor 12), respiration, as well as accelerometer values. One example of a similar method is described in Michele RD, Gatta G, Leo AD, Cortesi M, Andina F, Tam E, Boit MD, Merni F Estimation of the anaerobic threshold from heart rate variability in an incremental swimming test J Strength Cond Res. 2011 Dec 20

Alternatively, the anaerobic threshold may be calculated using the age of the user and the heart rate of the user, for example, a maximum heart rate may be given by the equation 220 - user age. The anaerobic threshold may then be calculated by calculating a percentage (say 85%) of this maximum heart rate. The threshold may be stored in a memory in the device and retrieved by the anaerobic threshold module 20. Any other suitable method may be used to estimate the anaerobic threshold.

The heart rate input by the ECG sensor can be compared to the estimated heart rate at the anaerobic threshold. If the heart rate input is greater than the estimated heart rate at the anaerobic threshold the anaerobic threshold module will determine that the anaerobic threshold has been passed. Conversely, if the heart rate input is less than the estimated heart rate at the anaerobic threshold the anaerobic threshold module will determine that the anaerobic threshold has not been passed

If the anaerobic threshold for the user has not been passed then the user is predominantly using aerobic metabolism and therefore the calculated physical activity intensity is an accurate estimate of the actual physical activity intensity and output to the display for the user to view.

If the anaerobic threshold for the user has been passed then the user is predominantly using anaerobic metabolism. In this instance the anaerobic threshold module 20 invokes a lactate estimator 22 to estimate the plasma lactate concentration from the estimated $VO_2$ value.

The plasma lactate concentration is estimated using any suitable known relationship between plasma lactate concentration and a physical feature of the user's body. For example, the plasma lactate concentration may be estimated using the $VO_2$ estimate using the following equations:

$$LA = 10^{(0.082 \lg VO_2 - 0.2)} \text{ where } VO_2 < 1.51$$

$$LA = 10^{(2.88 \lg VO_2 - 0.7)} \text{ where } VO_2 \geq 1.51$$

where LA is the estimated plasma lactate concentration.

(derived from W. L. Beaver, K.W., and B. J. Whipp, "Improved detection of lactate threshold during exercise using a log-log transformation". Applied Physiology, 1985. 59: p. 1936 - 1940)

The estimated plasma lactate concentration is then passed to a lactate to calorie convertor which converts the estimated plasma lactate concentration into calories. One possible method for converting lactate concentration into calories is described in R. Margaria, P.C., F. Mangili, "Balance and kinetics of anaerobic energy release during strenuous exercise in man". Applied Physiology, 1964. 19: p. 623 - 628. In this document the relationship between lactate concentration and calories was derived to be:

$$PAI_{anaerobic} = \frac{20LA}{0.75}$$

At the same time the estimated $VO_2$ value is also converted into calories to obtain an estimate of the calories associated with aerobic metabolism. One possible equation for estimating calories using $VO_2$ concentration is:

$$PAI_{aerobic} = 5.01 * VO_2$$

$PAI_{anaerobic}$ is combined with $PAI_{aerobic}$ to give a final compensated energy expenditure value which can be output to display.

In this way the user can be provided with an accurate estimate of energy expenditure when they are exercising beyond their aerobic capacity.

As will be understood by the skilled person the Physical Activity Intensity and $VO_2$ values provided by the Physical Activity Intensity and VO$_2$ estimator may be calculated using any suitable method provided the VO$_2$ and PAI are estimated as if the anaerobic threshold has not been breached i.e. assuming that the rate of increase in carbon dioxide is proportional to the increase in oxygen uptake. For example, the method described in US 2008/275348 may be used.

Although the present invention has been described with the Physical Activity Intensity and VO$_2$ values provided by the Physical Activity Intensity and VO$_2$ estimator being passed to an anaerobic threshold module the skilled person will understand that the anaerobic threshold module may use the input of the sensors to determine whether the anaerobic threshold has been passed concurrently with the Exercise Intensity and/or Physical Activity Intensity and VO$_2$ values being estimated. The output of the anaerobic threshold module may then be used to determine whether to output the Physical Activity Intensity and VO$_2$ values or pass the Physical Activity Intensity and VO$_2$ values to the anaerobic energy expenditure estimator with the anaerobic threshold module never receiving the Physical Activity Intensity and VO$_2$ values.

Alternatively, the anaerobic threshold module may use the input of the sensors to determine whether the anaerobic threshold has been passed before the Exercise Intensity and/or Physical Activity Intensity and VO$_2$ values being estimated. The output of the anaerobic threshold module may then be used to determine whether to use the aerobic model to estimate the Physical Activity Intensity and VO$_2$ values or pass the Physical Activity Intensity and VO$_2$ values to the anaerobic energy expenditure estimator with the anaerobic threshold module never receiving the Physical Activity Intensity and VO$_2$ values.

The skilled person will also understand that, although the present invention, describes the measurement of energy being calories, any suitable unit of energy may be calculated and output to the user.

The sensors may be any suitable sensor for monitoring characteristics of the user's body. For example, the sensor may be one or more of a heart rate monitor, an accelerometer and a gyroscope. The sensor may transmit recorded data using any suitable means. For example, the sensor may transmit the data wirelessly or through a wire connected to the device.

Additionally, the device may not only be provided with a display but additionally, or alternatively may be provided with any suitable means to output the calculated energy expenditure to a separate device. The device may be, for example, a personal computer, a remote server or any other suitable device.

Additionally, the device may be integrated into other devices. For example, it may form part of a user's cellular telephone.

## Claims

**1.** A device (10) comprising:

an input configured to receive data from at least one body sensor (12);
an aerobic processor configured to estimate aerobic energy expenditure of a user from the data using an aerobic metabolism model;
an anaerobic threshold module (20) configured to determine from the data whether the anaerobic threshold for the user has been exceeded;
an output configured to output the estimated aerobic energy expenditure if the anaerobic threshold for the user has not been exceeded;
an anaerobic energy expenditure estimator (22) configured to estimate anaerobic energy expenditure of the user due to anaerobic respiration from the data;
a processor configured to combine the estimated anaerobic energy expenditure and the estimated aerobic energy expenditure to produce a total energy expenditure when the anaerobic threshold for the user has been exceeded;
an output configured to output the total energy expenditure if the anaerobic threshold for the user has been exceeded,

wherein the aerobic processor comprises:

an exercise intensity classifier (16) configured to classify exercise of the user using data received by the input; and
an estimator (18) configured to estimate physical activity intensity and an estimated oxygen level in the respiratory gases according to the classification of the exercise wherein the output is configured to output the estimated physical activity intensity if the anaerobic threshold for the user has not been exceeded,

wherein the anaerobic energy expenditure (22) estimator comprises:

a lactate estimator configured to estimate plasma lactate concentration; and
a lactate to calorie convertor configured to convert the estimated plasma lactate concentration to a calorie value representing the anaerobic energy expenditure;
and the device further comprises:

an aerobic energy expenditure estimator configured to calculate a calorie value representing the aerobic energy expenditure using the estimated oxygen level in the respiratory gases;
a processor configured to add the calorie value representing the anaerobic energy expenditure and the calorie value representing the aerobic energy expenditure configured to provide an estimate of the total energy expenditure when the anaerobic threshold has been passed, and

wherein the plasma lactate concentration estimator is configured to estimate plasma lactate concentration using:

$$LA = 10^{(0.082\lg VO_2 - 0.2)} \text{ where } VO_2 < 1.51$$

$$LA = 10^{(2.88\lg VO_2 - 0.7)} \text{ where } VO_2 \geq 1.51$$

where LA is the plasma lactate concentration and wherein $VO_2$ is the estimated oxygen level in the respiratory gases.

2. A device (10) as claimed in claim 1 wherein the estimated plasma lactate concentration is converted to a calorie value representing the anaerobic energy expenditure using the equation:

$$PAI_{anaerobic} = \frac{20 LA}{0.75}$$

3. A device (10) as claimed in claim 1 or claim 2 wherein the aerobic energy expenditure estimator is configured to calculate the calorie value representing the aerobic energy expenditure using the following equation:

$$PAI_{aerobic} = 4.76 * VO_2$$

4. A device (10) as claimed in any one of the preceding claims wherein the body sensor comprises one or more of an ECG, an accelerometer and a gyroscope.

5. A device (10) as claimed in any one of the preceding claims wherein the data comprises one or more of heart rate, respiration rate, heart rate variability, fluctuations in the ECG R-R intervals, accelerometer measurements and gyroscope measurements.

6. A device (10) as claimed in any preceding claims wherein the input is configured to receive the data over a wireless connection.

7. A method comprising: at a device (10)

receiving data from at least one body sensor (12);
estimating aerobic energy expenditure of a user using an aerobic metabolism model;
determining from the data whether the anaerobic threshold for a user has been exceeded;
outputting the estimated aerobic energy expenditure if the anaerobic threshold for the user has not been exceeded;
estimating anaerobic energy expenditure due to anaerobic respiration if the anaerobic threshold of the user has been exceeded;
combining the estimated anaerobic energy expenditure and the estimated aerobic energy expenditure to produce a total energy expenditure if the anaerobic threshold for the user has been exceeded; and
outputting the total energy expenditure if the anaerobic threshold for the user has been exceeded,

wherein estimating aerobic energy expenditure using an aerobic metabolism model comprises:

classifying exercise of the user using data received by the input; and
estimating physical activity intensity and the estimated oxygen level in the respiratory gases according to the classification of the exercise wherein the output outputs the estimated physical activity intensity if the anaerobic threshold for the user has not been exceeded,

wherein estimating anaerobic energy expenditure due to anaerobic respiration comprises:

estimating plasma lactate concentration; and
converting the estimated plasma lactate concentration to a calorie value representing the anaerobic energy expenditure
and the method further comprises

calculating a calorie value representing the aerobic energy expenditure using the estimated oxygen level in the respiratory gases;
adding the calorie value representing the anaerobic energy expenditure and the calorie value representing the aerobic energy expenditure to provide an estimate of the total energy expenditure when the anaerobic threshold has been passed,and

wherein the plasma lactate concentration is estimated using:

$$LA = 10^{(0.082\,\lg VO_2 - 0.2)} \text{ where } VO_2 < 1.51$$

$$LA = 10^{(2.88\,\lg VO_2 - 0.7)} \text{ where } VO_2 \geq 1.51$$

where LA is the plasma lactate concentration.

8. A method as claimed in claim 7, comprising

converting the estimated plasma lactate concentration to a calorie value representing the anaerobic energy expenditure is performed using the equation:

$$PAI_{anaerobic} = \frac{20LA}{0.75}.$$

9. A method as claimed in claim 7 wherein calculating a calorie value representing the aerobic energy expenditure uses the following equation:

$$PAI_{aerobic} = 4.76 * VO_2$$

10. A method as claimed in any one of claims 7 to 9 wherein the data comprises one or more of heart rate, variability in the ECG R-R intervals, respiratory rate, accelerometer measurements and gyroscope measurements.

**Patentansprüche**

1. Vorrichtung (10), die Folgendes umfasst:

einen Eingang, der dazu ausgelegt ist, Daten von mindestens einem Körpersensor (12) zu empfangen;
einen aeroben Prozessor, der dazu ausgelegt ist, unter Verwendung eines aeroben Metabolismusmodells anhand der Daten einen aeroben Energieverbrauch eines Benutzers der Vorrichtung zu schätzen;
ein anaerobes Schwellwertmodul (20), das dazu ausgelegt ist, anhand der Daten zu bestimmen, ob der anaerobe Schwellwert des Benutzers überschritten wurde;

einen Ausgang, der dazu ausgelegt ist, den geschätzten aeroben Energieverbrauch auszugeben, wenn der anaerobe Schwellwert für den Benutzer nicht überschritten wurde;

einen anaeroben Energieverbrauchsschätzer (22), der dazu ausgelegt ist, anhand der Daten einen anaeroben Energieverbrauch des Benutzers infolge von anaerober Atmung zu schätzen;

einen Prozessor, der dazu ausgelegt ist, den geschätzten anaeroben Energieverbrauch und den geschätzten aeroben Energieverbrauch zu kombinieren, um einen Energiegesamtverbrauch zu produzieren, wenn der anaerobe Schwellwert für den Benutzer überschritten wurde;

einen Ausgang, der dazu ausgelegt ist, den Energiegesamtverbrauch auszugeben, wenn der anaerobe Schwellwert für den Benutzer überschritten wurde;

wobei der aerobe Prozessor Folgendes umfasst:

einen Übungsintensitätsklassifizierer (16), der dazu ausgelegt ist, eine Übung des Benutzers unter Verwendung der vom Eingang empfangenen Daten zu klassifizieren;

einen Schätzer (18), der dazu ausgelegt ist, eine physische Aktivitätsintensität und den geschätzten Sauerstoffpegel in den Atemgasen gemäß der Klassifizierung der Übung zu schätzen, wobei der Ausgang dazu ausgelegt ist, die geschätzte physische Aktivitätsintensität auszugeben, wenn der anaerobe Schwellwert für den Benutzer nicht überschritten wurde,

wobei der anaerobe Energieverbrauchsschätzer (22) Folgendes umfasst:

einen Laktatschätzer, der dazu ausgelegt ist, eine Plasmalaktatkonzentration zu schätzen; und

einen Laktat-Kalorien-Umwandler, der dazu ausgelegt ist, die geschätzte Plasmalaktatkonzentration in einen Kalorienwert umzuwandeln, der die anaerobe Energie repräsentiert.

und die Vorrichtung ferner Folgendes umfasst:

einen aeroben Energieverbrauchsschätzer, der dazu ausgelegt ist, unter Verwendung des geschätzten Sauerstoffpegels in den Atemgasen einen Wert zu berechnen, der den aeroben Energieverbrauch repräsentiert;

einen Prozessor, der dazu ausgelegt ist, den Kalorienwert, der die anaerobe Energie repräsentiert, und den Kalorienwert, der den aeroben Energieverbrauch repräsentiert, zu addieren, dazu ausgelegt, eine Schätzung des Energiegesamtverbrauchs bereitzustellen, wenn der anaerobe Schwellwert passiert wurde, und

wobei der Plasmalaktatkonzentrationsschätzer dazu ausgelegt ist, unter Verwendung von Folgendem eine Plasmalaktatkonzentration zu schätzen:

$$LA = 10^{(0,082 \, lg \, VO_2 \, -0.2)} \text{ wo } VO_2 < 1,51$$

$$LA = 10^{(2,88 \, lg \, VO_2 \, -0,7)} \text{ wo } VO_2 \geq 1,51$$

wo LA die Plasmalaktatkonzentration und wobei $VO_2$ der geschätzte Sauerstoffpegel in den Atemgasen ist.

2. Vorrichtung (10) nach Anspruch 1, wobei die geschätzte Plasmalaktatkonzentration unter Verwendung der folgenden Gleichung in einen Kalorienwert umgewandelt wird, der die anaerobe Energie repräsentiert:

$$PAI_{anaerobic} = \frac{20LA}{0,75}$$

3. Vorrichtung (10) nach Anspruch 1 oder 2, wobei der aerobe Energieverbrauchsschätzer dazu ausgelegt ist, unter Verwendung der folgenden Gleichung die Kalorien zu berechnen:

$$PAI_{aerobic} = 4,76 * VO_2$$

4. Vorrichtung (10) nach einem der vorhergehenden Ansprüche, wobei der Körpersensor eines oder mehrere von einem EKG, einem Beschleunigungsmesser und einem Gyroskop umfasst.

5. Vorrichtung (10) nach einem der vorhergehenden Ansprüche, wobei die Daten eines oder mehrere von einer Herzfrequenz, einer Atemrate, einer Herzfrequenzvariabilität, Schwankungen bei den EKG-R-R-Intervallen, Beschleunigungsmessermessungen und Gyroskopmessungen umfassen.

6. Vorrichtung (10) nach einem der vorhergehenden Ansprüche, wobei der Eingang dazu ausgelegt ist, Daten über eine drahtlose Verbindung zu empfangen.

7. Verfahren, das Folgendes umfasst: an einer Vorrichtung (10)
Empfangen von Daten von mindestens einem Körpersensor (12);
Schätzen eines Energieverbrauchs eines Benutzers der Vorrichtung unter Verwendung eines aeroben Metabolismusmodells;
Bestimmen anhand der Daten, ob der anaerobe Schwellwert für den Benutzer überschritten wurde;
Ausgeben des geschätzten Energieverbrauchs, wenn der anaerobe Schwellwert für den Benutzer nicht überschritten wurde;
Schätzen eines Energieverbrauchs infolge von anaerober Atmung;
Kombinieren des geschätzten anaeroben Energieverbrauchs und des geschätzten aeroben Energieverbrauchs, um einen Energiegesamtverbrauch zu produzieren, wenn der anaerobe Schwellwert für den Benutzer überschritten wurde; und
Ausgeben des Energiegesamtverbrauchs, wenn der anaerobe Schwellwert für den Benutzer überschritten wurde, wobei das Schätzen eines Energieverbrauchs unter Verwendung eines aeroben Metabolismusmodells Folgendes umfasst:

Klassifizieren einer Übung des Benutzers unter Verwendung der vom Eingang empfangenen Daten und Schätzen einer physischen Aktivitätsintensität und des geschätzten Sauerstoffpegels in den Atemgasen gemäß der Klassifizierung der Übung, wobei der Ausgang die geschätzte physische Aktivitätsintensität ausgibt, wenn der anaerobe Schwellwert für den Benutzer nicht überschritten wurde, wobei das Schätzen eines Energieverbrauchs infolge von anaerober Atmung Folgendes umfasst:

Schätzen einer Plasmalaktatkonzentration und
Umwandeln der geschätzten Plasmalaktatkonzentration in einen Kalorienwert, der die anaerobe Energie repräsentiert

und das Verfahren ferner Folgendes umfasst
Berechnen eines Kalorienwerts, der den aeroben Energieverbrauch repräsentiert, unter Verwendung des geschätzten Sauerstoffpegels in den Atemgasen;
Addieren des Kalorienwerts, der die anaerobe Energie repräsentiert, und des Kalorienwerts, der den aeroben Energieverbrauch repräsentiert, um eine Schätzung des Energiegesamtverbrauchs bereitzustellen, wenn der anaerobe Schwellwert passiert wurde, und wobei die Plasmalaktatkonzentration geschätzt wird unter Verwendung von:

$$LA = 10^{(0,082 \lg VO_2 -0,2)} \text{ wo } VO_2 < 1,51$$

$$LA = 10^{(2,88 \lg VO_2 -0,7)} \text{ wo } VO_2 \geq 1,51$$

wo LA die Plasmalaktatkonzentration und wobei $VO_2$ der geschätzte Sauerstoffpegel in den Atemgasen ist.

8. Verfahren nach Anspruch 7, das ferner Folgendes umfasst Umwandeln der geschätzten Plasmalaktatkonzentration in einen Kalorienwert, der die anaerobe Energie repräsentiert unter Verwendung der folgenden Gleichung durchgeführt wird:

$$PAI_{\text{anaerobic}} = \frac{20LA}{0,75}$$

**9.** Verfahren nach Anspruch 7, wobei das Berechnen eines Kalorienwerts, der den aeroben Energieverbrauch repräsentiert, die folgende Gleichung verwendet:

$$PAI_{\text{aerobic}} = 4{,}76 * VO_2$$

**10.** Verfahren nach einem der Ansprüche 7 bis 9, wobei die Daten eines oder mehrere von einer Herzfrequenz, einer Variabilität bei den EKG-R-R-Intervallen, einer Atemrate, Beschleunigungsmessermessungen und Gyroskopmessungen umfassen.

**Revendications**

**1.** Dispositif (10), comprenant :

une entrée configurée pour recevoir des données en provenance d'au moins un capteur corporel (12) ;
un processeur aérobie configuré pour estimer une dépense énergétique aérobie d'un utilisateur du dispositif à partir desdites données à l'aide d'un modèle de métabolisme aérobie ;
un module de seuil anaérobie (20) configuré pour déterminer, à partir desdites données, si le seuil anaérobie pour l'utilisateur a été dépassé ;
une sortie configurée pour fournir en sortie la dépense énergétique aérobie estimée si le seuil anaérobie pour l'utilisateur n'a pas été dépassé ;
un estimateur de dépense énergétique anaérobie (22) configuré pour estimer une dépense énergétique anaérobie de l'utilisateur suite à une respiration anaérobie à partir desdites données ;
un processeur configuré pour combiner la dépense énergétique anaérobie estimée et la dépense énergétique aérobie estimée afin de produire une dépense énergétique totale lorsque le seuil anaérobie pour l'utilisateur a été dépassé ;
une sortie configurée pour fournir en sortie la dépense énergétique totale si le seuil anaérobie pour l'utilisateur a été dépassé,
dans lequel le processeur aérobie comprend :

un classificateur d'intensité d'exercice (16) configuré pour classer l'exercice de l'utilisateur à l'aide des données reçues par l'entrée ;
un estimateur (18) configuré pour estimer une intensité d'activité physique et le niveau d'oxygène estimé dans les gaz respiratoires en fonction de la classification de l'exercice, dans lequel la sortie est configurée pour fournir en sortie l'intensité d'activité physique estimée si le seuil anaérobie pour l'utilisateur n'a pas été dépassé,
dans lequel l'estimateur de dépense énergétique anaérobie (22) comprend :

un estimateur de lactate configuré pour estimer la concentration plasmatique en lactate ; et
un convertisseur lactate vers calorie configuré pour convertir la concentration plasmatique en lactate estimée en une valeur en calories représentant l'énergie anaérobie ;

et le dispositif comprend en outre :

un estimateur de dépense énergétique aérobie configuré pour calculer une valeur représentant la dépense énergétique aérobie à l'aide du niveau d'oxygène estimé dans les gaz respiratoires ;
un processeur configuré pour additionner la valeur en calories représentant l'énergie anaérobie et la valeur en calories représentant la dépense énergétique aérobie et configurée pour fournir une estimation de la dépense énergétique totale lorsque le seuil anaérobie a été dépassé, et
dans lequel l'estimateur de concentration plasmatique en lactate est configuré pour estimer la concentration plasmatique en lactate à l'aide des équations ci-dessous :

$$LA = 10^{(0{,}082 \lg VO_2 - 0{,}2)} \text{ où } VO_2 < 1{,}51$$

$$LA = 10^{(2{,}88 \lg VO_2 - 0{,}7)} \text{ où } VO_2 \geq 1{,}51$$

LA représentant la concentration plasmatique en lactate et $VO_2$ représentant le niveau d'oxygène estimé dans les gaz respiratoires.

2. Dispositif (10) selon la revendication 1, dans lequel la concentration plasmatique en lactate estimée est convertie en une valeur en calories représentant l'énergie anaérobie, à l'aide de l'équation ci-dessous :

$$PAI_{anaerobie} = \frac{20LA}{0,75}$$

3. Dispositif (10) selon la revendication 1 ou 2, dans lequel l'estimateur de dépense énergétique aérobie est configuré pour calculer les calories à l'aide de l'équation ci-dessous :

$$PAI_{aérobie} = 4,76 * VO_2$$

4. Dispositif (10) selon l'une quelconque des revendications précédentes, dans lequel le capteur corporel comprend un ou plusieurs parmi un ECG, un accéléromètre et un gyroscope.

5. Dispositif (10) selon l'une quelconque des revendications précédentes, dans lequel les données comprennent une ou plusieurs parmi une fréquence cardiaque, une fréquence respiratoire, une variabilité de fréquence cardiaque, des fluctuations au sein des intervalles R-R d'ECG, des mesures accélérométriques et des mesures gyroscopiques.

6. Dispositif (10) selon l'une quelconque des revendications précédentes, dans lequel l'entrée est configurée pour recevoir les données par l'intermédiaire d'une connexion sans fil.

7. Procédé comprenant les étapes consistant à :

au niveau d'un dispositif (10)
recevoir des données en provenance d'au moins un capteur corporel (12) ;
estimer une dépense énergétique d'un utilisateur du dispositif à l'aide d'un modèle de métabolisme aérobie ;
déterminer à partir desdites données si le seuil anaérobie pour l'utilisateur a été dépassé ;
fournir la dépense énergétique estimée si le seuil anaérobie pour l'utilisateur n'a pas été dépassé ;
estimer la dépense énergétique suite à une respiration anaérobie ;
combiner la dépense énergétique anaérobie estimée et la dépense énergétique aérobie estimée afin de produire une dépense énergétique totale lorsque le seuil anaérobie pour l'utilisateur a été dépassé ; et
fournir la dépense énergétique totale si le seuil anaérobie pour l'utilisateur a été dépassé,
dans lequel l'étape d'estimation de la dépense énergétique à l'aide d'un modèle de métabolisme aérobie comprend les étapes consistant à :

classer l'exercice de l'utilisateur à l'aide de données reçues par l'entrée ; et
estimer une intensité d'activité physique et le niveau d'oxygène estimé dans les gaz respiratoires en fonction de la classification de l'exercice, dans lequel la sortie fournit l'intensité d'activité physique estimée si le seuil anaérobie pour l'utilisateur n'a pas été dépassé,

dans lequel l'étape d'estimation de la dépense énergétique suite à une respiration anaérobie comprend les étapes consistant à :

estimer une concentration plasmatique en lactate ; et
convertir la concentration plasmatique en lactate estimée en une valeur en calories représentant l'énergie anaérobie

et le procédé comprend en outre les étapes consistant à
calculer une valeur en calories représentant la dépense énergétique aérobie à l'aide du niveau d'oxygène estimé dans les gaz respiratoires ;
ajouter la valeur en calories représentant l'énergie anaérobie et la valeur en calories représentant la dépense énergétique aérobie afin de fournir une estimation de la dépense énergétique totale lorsque le seuil anaérobie a été dépassé, et dans lequel la concentration plasmatique en lactate est estimée à l'aide des équations ci-

dessous :

$$LA = 10^{(0,082\lg VO_2 - 0,2)} \text{ où } VO_2 < 1,51$$

$$LA = 10^{(2,88\lg VO_2 - 0,7)} \text{ où } VO_2 \geq 1,51$$

LA représentant la concentration plasmatique en lactate et $VO_2$ représentant le niveau d'oxygène estimé dans les gaz respiratoires.

**8.** Procédé selon la revendication 7, comprenant une étape consistant à convertir la concentration plasmatique en lactate estimée en une valeur en calories représentant l'énergie anaérobie réalisée à l'aide de l'équation ci-dessous :

$$PAI_{anaérobie} = \frac{20LA}{0,75}$$

**9.** Procédé selon la revendication 7, dans lequel l'étape de calcul d'une valeur en calories représentant la dépense énergétique aérobie utilise l'équation ci-dessous :

$$PAI_{aérobie} = 4,76*VO_2$$

**10.** Procédé selon l'une quelconque des revendications 7 à 9, dans lequel les données comprennent une ou plusieurs parmi une fréquence cardiaque, une variabilité dans les intervalles R-R d'ECG, une fréquence respiratoire, des mesures accélérométriques et des mesures gyroscopiques.

Figure 1

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 6554776 B
- US 7470234 B
- US 7648463 B
- US 2004186390 A1
- EP 1618844 A1
- US 2008275348 A

**Non-patent literature cited in the description**

- **A. ARDEVOL et al.** *European Journal of Physiology,* 01 February 1998, 495-502
- **MICHELE RD ; GATTA G ; LEO AD ; CORTESI M ; ANDINA F ; TAM E ; BOIT MD ; MERNI F.** Estimation of the anaerobic threshold from heart rate variability in an incremental swimming test. *J Strength Cond Res.,* 20 December 2011
- **W. L. BEAVER, K.W. ; B. J. WHIPP.** Improved detection of lactate threshold during exercise using a log-log transformation. *Applied Physiology,* 1985, vol. 59, 1936-1940
- **R. MARGARIA ; P.C., F. MANGILI.** Balance and kinetics of anaerobic energy release during strenuous exercise in man. *Applied Physiology,* 1964, vol. 19, 623-628